# EUROPÄISCHE PATENTSCHRIFT

(11) **EP 2 922 463 B1**
(45) Veröffentlichungstag und Bekanntmachung des Hinweises auf die Patenterteilung: **14.03.2018**
(21) Anmeldenummer: 13802875.8
(22) Anmeldetag: 15.11.2013
(51) Int. Cl.: A61B 5/00, A61B 5/12, G10K 11/178

(54) **MESSUNG OTOAKUSTISCHER EMISSIONEN MITTELS AKTIVER SCHALLDÄMPFUNG**
MEASURING OTOACOUSTIC EMISSIONS BY MEANS OF ACTIVE SOUND ABSORPTION
MESURE D'OTOÉMISSIONS ACOUSTIQUES AU MOYEN D'UNE ATTÉNUATION ACTIVE DU BRUIT

(30) Priorität: 21.11.2012 DE 102012022795
(43) Veröffentlichungstag der Anmeldung: 30.09.2015
(73) Patentinhaber: Thie, Rainer, 15827 Blankenfelde-Mahlow (DE)
(72) Erfinder: BALKENHOL, Tobias, 68163 Mannheim (DE); DELB, Wolfgang, 67663 Kaiserslautern (DE); THIE, Rainer, 15827 Blankenfelde (DE)
(74) Vertreter: Müller-Boré & Partner Patentanwälte PartG mbB
(86) Internationale Anmeldenummer: PCT/EP2013/003446
(87) Internationale Veröffentlichungsnummer: WO 2014/079549

(56) Entgegenhaltungen:
- EP-A1- 1 465 117
- WO-A1-2008/139404
- CH-A5- 693 664
- MATTHEW A. BROMWICH ET AL: "Active Noise Reduction Audiometry: A Prospective Analysis of a New Approach to Noise Management in Audiometric Testing", THE LARYNGOSCOPE, Bd. 118, Nr. 1, 1. Januar 2008 (2008-01-01), Seiten 104-109, XP055099710, ISSN: 0023-852X, DOI: 10.1097/MLG.0b013e31815743ac

## Beschreibung

Die Erfindung betrifft eine Sonde zur Messung von otoakustischer Emission.
Otoakustische Emissionen, kurz OAE, sind Schallwellen, die im Innenohr entstehen und über Gehörknöchelchen und Trommelfell in den äußeren Gehörgang gelangen und dort mit einem Messmikrophon aufgenommen werden können. Diese Emissionen werden durch die äußeren Haarzellen des Corti-Organs erzeugt, die beim aktiven Prozess der nichtlinearen Verstärkung durch kleinste Schwingungen der Basilarmembran zu verstärkten Eigenschwingungen angeregt werden. Die Eigenschwingung der äußeren Haarzellen lassen sich durch äußere Schallreize anregen (evozierte OAE) oder können spontan entstehen. Die spontanen OAE besitzen keine diagnostische Bedeutung, wohingegen die evozierten OAE für einen objektiven Test der kochleären Funktion verwendet werden.
Kommen kurze Schallimpulse in Form von akustischen Klicks, kurzen Tonimpulsen oder Chirps zum Einsatz, spricht man von Transitorisch-Evozierten-Otoakustischen-Emissionen (TEOAE), während Distorsionsprodukte-Otoakustische-Emissionen (DPOAE) mit zwei simultan applizierten Sinustönen unterschiedlicher Frequenz evoziert werden.
Im Neugeborenen-, Säuglings- und Kindesalter haben sich TEOAE als besonders geeignet für das Screening der kochleären Funktion erwiesen und sind beispielsweise in universellen Neugeborenen-Hörscreenings (NHS oder NGHS) als wichtiges Instrument zur Früherkennung kindlicher Hörstörungen anerkannt und vorgeschrieben. Solche Hörtests finden bei Neugeborenen in den ersten Lebenstagen direkt auf Säuglingsstationen statt. Zur Hörprüfung in Klinik und Praxis kommen dagegen sowohl TEOAE als auch DPOAE zum Einsatz, um genauere und eventuell frequenzspezifischere Informationen bei Innenohrhörschäden zu erlangen.

Die Messung der OAE findet häufig in geräuschvoller Umgebung, wie zum Beispiel auf Säuglingsstationen, in Räumen mit Klimaanlage oder sonstigen unerwünschten Hintergrundgeräuschen, statt. Umgebungslärm beeinflusst die Genauigkeit mit der OAE gemessen werden können, da der Signal-Stör-Abstand sinkt. Dadurch reduziert sich die sogenannte Spezifität für die Erkennung von Schwerhörigkeiten. Die Spezifität beschreibt die Fähigkeit eines Tests, möglichst nur Hörgestörte zu detektieren. Die Spezifität wird durch das Verhältnis aus Probanden, bei denen gemäß der Auswertung von OAE keine Hörstörung vorliegt und die tatsächlich normal hören, zu der Gesamtzahl aller getesteten normalhörigen Probanden beschrieben. Die Gesamtzahl setzt sich aus der Anzahl fälschlicherweise als auffällig eingestuften Normalhörenden und den richtig eingestuften Normalhörenden zusammen.

Insbesondere ist die Messung der OAE im Tieftonbereich für Frequenzen f < 1 kHz auch unter normalen Messbedingungen infolge von Störgeräuschen, wie z.B. Umgebungslärm, so schwerwiegend beeinflusst, dass eine Messung im Tieftonbereich üblicherweise nicht durchgeführt wird.

Untersuchungen haben gezeigt, dass bei einem durchschnittlichen Geräuschpegel am Bett eines Neugeborenen von ca. 60 dB(A) eine Spezifität von etwa 97% erreicht werden kann, während sich diese oberhalb von 70 dB(A) auf nur noch ca. 4% reduziert (H. Salina, A. Abdullah, S. Mukari, M. T. Azmi, "Effects of background noise on recording of portable transient-evoked otoacoustic emission in newborn hearing screening", European Archives of Oto-Rhino-Laryngology 267, S. 495-499, 2010). Der Einheit dB(A) liegt eine A-Gewichtung des Lautstärkepegels im Frequenzbereich zu Grunde.

Für andere audiologische Anwendungen, jedoch nicht für die Messung von OAE, wurden Konzepte entwickelt, bei denen subjektive Hörprüfungen mit einem zirkumauralen, geschlossenen Kopfhörer durchgeführt werden. In dem Kopfhörer können passive Schalldämpfungen mit Schallfiltern integriert sein oder Vorrichtungen zur aktiven Schalldämpfung (U.S. Pat. No. 6,532,296 B1, U.S. Pat. No. 6,396,930 B1), das heißt zur Aussendung von Gegenschall zum destruktiven Interferieren und somit zur Reduktion des Einflusses von Umgebungslärm auf die Hörprüfung.

Dokument CH 693 664 A5 betrifft eine Vorrichtung und ein Verfahren zur OAE Messung. Dabei werden Störgeräusche über ein Referenzmikrofon aufgenommen, durch einen adaptiven Filter gefiltert, und anschließend vom gestörten Messsignal subtrahiert.

Dokument WO 2008/139404 A1 betrifft ein Audiometer mit einem externen Kopfhörer. Das Audiometer ist als Multi-Funktions-Audiometer ausgebildet und kann u.A. zur Messung von OAE verwendet werden.

Für Neugeborenen-Hörscreenings sind solche Vorrichtungen ungeeignet, da sich eine Positionierung eines Schallschutzkopfhörers als problematisch gestaltet. Aufgrund der filigranen Anatomie eines Säuglingskopfes kann eine sichere und für den Säugling schmerzfreie und demnach nicht störende und irritierende Ankopplung des Kopfhörers nicht gewährleistet werden. Zudem ist durch das Aufsetzen eines Schallschutzkopfhörers beim Neugeborenen/Patienten ein Verrutschen der Messapparate möglich, so dass ein wiederholtes Positionieren der Vorrichtung nötig ist, um den Messprozess durchzuführen.

Gemäß einem Aspekt liegt der Erfindung die Aufgabe zugrunde, eine Vorrichtung und ein Verfahren bereitzustellen, um die Messung von OAE zu verbessern, insbesondere um OAE auch in geräuschvoller Umgebung und mit einem daraus resultierenden geringen Signal-Stör-Abstand mit einer möglichst hohen Spezifität messen zu können.

Diese Aufgabe wird durch eine Sonde gemäß Anspruch 1 gelöst. Bevorzugte Ausführungsformen sind Gegenstände der abhängigen Ansprüche.

Demnach weist die Sonde zur Messung otoakustischer Emission einen Sondenkörper auf, wobei der Sondenkörper so ausgebildet ist, dass er teilweise entlang einer Einführrichtung in einen Gehörgang eines Probanden bzw. Patienten einführbar ist. Der Sondenkörper weist auf:
- ein Anregungsmittel zur Anregung otoakustischer Emission,
- ein Messmittel zur Messung der otoakustischen Emission und
- ein Dämpfungsmittel zur aktiven Schalldämpfung.

Ein Proband ist dabei eine Person beliebigen Alters. Insbesondere kann ein Proband ein Neugeborenes sein. Die Sonde kann aber auch zur Messung der OAE bei älteren Personen ausgebildet sein.

Unter dem Begriff Sonde kann eine im Wesentlichen stabförmige und/oder zylinderförmige Vorrichtung verstanden werden, die zumindest teilweise in den menschlichen Körper einführbar ist. Die Längsachse der zylinderförmigen Sonde kann dabei mit dem Richtungsvektor der Einführrichtung zusammenfallen.

Im Wesentlichen stabförmig bzw. zylinderförmig bedeutet dabei, dass die Sonde länglich ausgebildet ist, so dass sie mit einem Ende in den Gehörgang eines Ohres eingeführt und gleichzeitig am gegenüberliegenden Ende von einer Person, die die Messung durchführen soll, festgehalten und im Gehörgang positioniert werden kann. Dabei kann die Sonde so filigran ausgebildet sein, dass sie über Ohrstöpsel ebenfalls in den Gehörgang eines Neugeborenen einführbar ist.

Der Sondenkörper kann ein oder mehrteilig ausgebildet sein und aus einer oder mehreren Gehäusekammern bestehen. Der Sondenkörper bildet ein Multifunktionsbauteil, das sowohl Mittel zur Anregung und Messung von OAE aufweist, als auch ein Dämpfungsmittel und Sensorik für die aktive Schalldämpfung im oder am selben Sondenkörper bereitstellt.

Die Formulierung "ein" Anregungsmittel, Messmittel bzw. Dämpfungsmittel ist dabei so zu verstehen, dass die Sonde zumindest eines dieser Mittel aufweist. Die Sonde kann auch mehrere dieser Mittel aufweisen.

Die Ankopplung an den Gehörgang des Probanden kann über austauschbare und elastisch komprimierbare Ohrstöpsel erfolgen, die je nach Größe des Gehörgangs im unkomprimierten Zustand üblicherweise einen Durchmesser im Bereich von ca. 3mm bis 16mm sowie eine Länge von ca. 8mm bis 12mm aufweisen können.

Der Durchmesser des auswählbaren Ohrstöpsels richtet sich nach der Größe des Gehörgangseingangs von dem Probanden, so dass eine schmerzfreie und akustisch dichte Ankopplung an den Gehörgang ermöglicht wird. Die Qualität der akustischen Dichtigkeit kann von einer Auswerteeinheit gemessen und der Person, die die Messung durchführt, angezeigt werden. Einführtiefen des Ohrstöpsels können im Bereich von ca. 4mm bis 10mm liegen.

An dem Ende der Sonde, das in den Gehörgang einführbar ist, sind ein Anregungsmittel und ein Messmittel für OAE angeordnet. Als Anregungsmittel für eine TEOAE kommt ein Lautsprecher zum Einsatz, während das Anregungsmittel für eine DPOAE zumindest zwei Lautsprecher aufweist, so dass die beiden zu applizierenden Sinustöne jeweils über separate Lautsprecher erzeugt werden. Der oder die Lautsprecher können als Miniaturlautsprecher ausgeführt sein. Als Messmittel kann zur Erfassung der TEOAE bzw. DPOAE ein Mikrophon verwendet werden, das als Miniaturmikrophon ausgeführt sein kann. Die akustische Ankopplung der Anregungsmittel und Messmittel an den Gehörgang kann über Sondenkanäle erfolgen.

Die Sonde weist weiterhin ein Dämpfungsmittel zur aktiven Schalldämpfung auf. Durch das Dämpfungsmittel kann der Umgebungslärm im Bereich der Sonde gedämpft werden. Das Prinzip der aktiven Schalldämpfung basiert auf der Berechnung eines Gegenschallsignals mit Hilfe einer Sensorik für die aktive Schalldämpfung. Das Gegenschallsignal wird durch das Dämpfungsmittel, welches auch als sekundäre Schallquelle bezeichnet wird, abgestrahlt und überlagert sich am gewünschten Ort der Lärmminderung destruktiv mit dem Umgebungslärm (Störgeräuschen), den primären Schallquellen. Das Dämpfungsmittel dient somit zur Erzeugung eines Gegenschalls, der auf dem Umgebungslärm abgestimmt sein kann. Damit interferieren die Schallwellen des vom Dämpfungsmittel ausgesandten Gegenschalls destruktiv mit den Schallwellen des Umgebungslärms.

Der Gegenschall kann so berechnet werden, dass sich als gewünschter Ort der Lärmminderung z.B. der Gehörgangseingang ergibt, wo der Gegenschall sich destruktiv mit dem Umgebungsschall überlagert. Damit wird der Umgebungslärm zumindest weitgehend reduziert. Die Anregung und Messung der otoakustischen Emission findet an dem Teil der Sonde statt, die in Messposition in den Gehörgang eingeführt ist.

Die Sensorik zur aktiven Schalldämpfung kann aus einem oder mehreren Mikrophonen bestehen. Das oder die Mikrophone können entweder in den Sondenkörper integriert oder an dem Sondenkörper befestigt sein.

Das Dämpfungsmittel kann einen Lautsprecher zum Aussenden von Gegenschall aufweisen oder als Lautsprecher ausgebildet sein. Der Lautsprecher kann in dem oder am Sondenkörper so ausgerichtet sein, dass er in der Messposition der Sonde den Gegenschall in Richtung zum Gehörgangseingang sendet. Die Messposition der Sonde ist der Zustand, in dem die Sonde zumindest teilweise in einen Gehörgang eines Probanden eingeführt ist. Das Dämpfungsmittel kann dabei einen oder mehrere Lautsprecher aufweisen. Der oder die Lautsprecher können entweder in den Sondenkörper integriert oder an dem Sondenkörper befestigt sein.

Gemäß einem Ausführungsbeispiel weist der Sondenkörper einen Einführbereich auf, der zum Einführen in einen Gehörgang ausgebildet ist, und einen externen Sondenbereich, der dazu ausgebildet ist, beim Messen otoakustischer Emissionen außerhalb des Gehörgangs angeordnet zu sein. Obwohl der Sondenkörper als einziges Multifunktionsbauteil ausgebildet ist, weist er zumindest den Einführbereich und den externen Sondenbereich auf. Der Einführbereich kann als Ohrstöpsel ausgebildet sein. Dabei kann das Anregungsmittel und das Messmittel im oder am Einführbereich angeordnet sein, z.B. in einer ersten Gehäusekammer des Sondenkörpers. Über Sondenkanäle, die am Anregungsmittel und Messmittel angekoppelt sein können, kann die Anregung und Messung der OAE im Inneren des Gehörgangs im oder am Einführbereich erfolgen. Das Dämpfungsmittel für die aktive Schalldämpfung kann Referenzmikrophon(e), Lautsprecher und Fehlermikrophon(e) aufweisen und im oder am äußeren Sondenbereich angeordnet sein.

Dabei erfolgt die Messung der otoakustischen Emission im Einführbereich, während im externen Sondenbereich die aktive Schalldämpfung wirkt. Der Einführbereich kann die Anregungsmittel und Messmittel zur Messung der OAE enthalten. Die Dämpfungsmittel für die aktive Schalldämpfung können im oder am externen Sondenbereich angeordnet sein, insbesondere in einer separaten Gehäusekammer. Dabei kann die Sonde zumindest zwei Gehäusekammern aufweisen. Eine erste Gehäusekammer kann diejenigen Komponenten enthalten, die zur Anregung und Messung der OAE dienen, wie das/die Anregungsmittel und die/das Messmittel. Die zweite Gehäusekammer kann das/die Dämpfungsmittel zur aktiven Schalldämpfung enthalten. Durch die Anordnung der Komponenten in zwei unterschiedlichen Gehäusekammern der Sonde wird eine unerwünschte Wechselwirkung der Komponenten untereinander reduziert. Beide Gehäusekammern sind Bestandteil ein und derselben Sonde und benachbart zueinander angeordnet.

Gemäß einem Ausführungsbeispiel sind an der Sonde zumindest ein Lautsprecher zum Aussenden von Gegenschall und zumindest ein Fehlermikrophon zur Überprüfung der aktiven Schalldämpfung, insbesondere zur Messung eines resultierenden Schalldrucks so angeordnet, dass zum Fehlermikrophon und/oder zum Gehörgangseingang hin propagierender Schall (Umgebungslärm) aktiv gedämpft wird. Diese Komponenten für die aktive Schalldämpfung können insbesondere in Einführrichtung hintereinander angeordnet sein.

Dabei kann die Sonde zumindest ein Referenzmikrophon zur Messung von Umgebungslärm aufweisen, wobei das Fehlermikrophon den resultierenden Schalldruck misst, der sich aus der Überlagerung des Umgebungslärms und des Gegenschalls ergibt.

Das Referenzmikrophon ist am weitesten von dem Ende der Sonde entfernt, das in einen Gehörgang einführbar ausgebildet ist. Das Referenzmikrophon misst den Schall aus der Umgebung, den sogenannten Umgebungslärm. Die Sonde kann ein oder mehrere Referenzmikrophone und ein oder mehrere Lautsprecher zur Aussendung von Gegenschall aufweisen. Der oder die Lautsprecher sind in Messposition der Sonde näher am Gehörgangseingang positioniert als das oder die Referenzmikrophone. Bevorzugt ist dabei die Laufzeit des Schalls vom Referenzmikrophon zum Ort der destruktiven Schallüberlagerung (beispielsweise Position des Fehlermikrophons) größer als das Zeitintervall, das die Auswerteeinheit und der Lautsprecher benötigen, um intern den Gegenschall zu berechnen und auszusenden. Dabei ist der Gegenschall auf den Umgebungslärm abgestimmt, so dass sie sich destruktiv überlagern.

Um die Überlagerung aus Umgebungslärm und Gegenschall am gewünschten Ort der Lärmminderung zu messen, kann zumindest ein Fehlermikrophon im oder am Sondenkörper angeordnet sein. Das Fehlermikrophon befindet sich in Messposition der Sonde näher am Gehörgangseingang als der Lautsprecher.

Der Lautsprecher und/oder das Referenzmikrophon können im oder am externen Sondenbereich angeordnet sein. Das Fehlermikrophon kann im oder am externen Sondenbereich angeordnet sein, oder unmittelbar benachbart zum Messmittel im Inneren der Sonde. Referenzmikrophon, Lautsprecher und Fehlermikrophon sind in Einführrichtung beabstandet voneinander an der Sonde angeordnet. Dabei ist mit der Position des Fehlermikrophons nicht das eigentliche elektrische Mikrophonbauteil gemeint, sondern der Ort, an dem das Fehlermikrophon Schall detektiert. Das Fehlermikrophon kann auch weiter im Inneren der Sonde angeordnet sein, wobei es zum Beispiel über eine Schallführung bzw. einen Sondenkanal an einem Ort im Bereich des Gehörgangseingangs so verbunden ist, dass es den Schall an diesem Ort detektiert.

Die aktive Schalldämpfung greift nicht in das Schallfeld im Gehörgang ein, sondern dämpft den Umgebungslärm im äußeren Bereich vor dem Gehörgangseingang auf der Grundlage einer gerichteten Abstrahlung des Gegenschalls. Zumindest ein Referenzmikrophon, zumindest ein Lautsprecher und zumindest ein Fehlermikrophon können in einem Bereich der Sonde angeordnet sein, der in Messposition der Sonde außerhalb des Gehörgangs angeordnet ist. Damit wirkt die aktive Schalldämpfung im Raumbereich vor dem Gehörgangseingang und beeinflusst nicht die Messung der OAE im Gehörgang.

Die OAE treten nur im Gehörgang auf und werden daher nicht in die Berechnung des Gegenschalls einbezogen, da sie von dem beabstandet positionierten Fehler- und Referenzmikrophonen nicht erfasst werden. Da die Interaktion zwischen Umgebungslärm und Gegenschall außerhalb des Gehörgangs erfolgt, ist eine direkte Wirkung des Gegenschalls auf die OAE ausgeschlossen oder zumindest gedämpft.

Gemäß einer Ausführungsform ist zumindest ein Fehlermikrophon zur aktiven Schalldämpfung an der Sonde so angeordnet, dass das Fehlermikrophon oder ein Fehlermikrophoneingang eines Fehlermikrophonkanals, der an das Fehlermikrophon gekoppelt ist, im Einführzustand bzw. in Messposition der Sonde in einem Raumbereich vor dem Gehörgangseingang angeordnet ist. Bei der aktiven Schalldämpfung bestimmt die Anordnung des Fehlermikrophons bzw. des Fehlermikrophoneingangs des Fehlermikrophonkanals den Ort, an dem die höchste aktive Schalldämpfung zu erwarten ist.

Gemäß einer Ausführungsform sind das Anregungsmittel und das Messmittel dazu ausgebildet, otoakustische Emissionen mit Frequenzen kleiner als 1 kHz zu messen. Dazu sind das Dämpfungsmittel und die Sensorik zur aktiven Schalldämpfung dazu ausgebildet, Umgebungslärm im Bereich von ca. 0,5kHz bis 2kHz aktiv zu dämpfen, so dass die Messung der OAE auch unterhalb von 1 kHz erfolgen kann. Unterhalb von 1 kHz ist die Messung der OAE gegenüber Umgebungslärm störanfällig, so dass bisher auf entsprechende Messungen verzichtet wurde. Durch eine solche aktive Schalldämpfung wird eine Messung der OAE in diesem Tieftonbereich ermöglicht. Die Sonde kann so ausgebildet sein, dass sie nicht auf die Messung in diesem Tieftonbereich beschränkt ist, sondern zusätzlich auch in einem höheren Frequenzbereich die Messung der OAE durchführen kann.

Gemäß einer Ausführungsform ist am Sondenkörper zumindest eine Schallführung so angeordnet, dass vom Dämpfungsmittel ausgesandter Gegenschall im Wesentlichen in Einführrichtung geführt wird. Eine solche Schallführung kann zum Beispiel durch eine Röhre oder einen hornförmigen Trichter erfolgen. An die Schallführung ist ein Lautsprecher des Dämpfungsmittels gekoppelt. Die Führung des Gegenschalls erfolgt dabei in Richtung zum Gehörgangseingang, wo auch zumindest ein Fehlermikrophon angeordnet sein kann.

Die Schallführung konzentriert den Schall und vermindert ein Abschwächen des Schalls auf dem Weg vom Dämpfungsmittel zum bevorzugten Ort der aktiven Schallauslöschung.

Gemäß einem Ausführungsbeispiel ist am Sondenkörper ein Griffbereich ausgebildet, an dem die Sonde beim Messen otoakustischer Emission festhaltbar ist.

Gemäß einer Ausführungsform ist ein Referenzmikrophon zur Messung von Umgebungslärm an einem schalldurchlässigen Mikrophonhalter angeordnet. Dabei kann der schalldurchlässige Mikrophonhalter im oder am externen Sondenbereich angeordnet sein. Das Referenzmikrophon dient zur Messung des Umgebungslärms. Zumindest ein Referenzmikrophon ist dazu vom Sondenkörper beabstandet positioniert. Der Mikrophonhalter ist schalldurchlässig ausgebildet, insbesondere schalldurchlässig für den Umgebungslärm, so dass der Mikrophonhalter das Schallfeld des Umgebungslärms nur geringfügig beeinflusst. Der Mikrophonhalter kann dabei am Sondenkörper angeordnet sein.

Dabei kann der Mikrophonhalter schalenförmig ausgebildet sein und am externen Sondenbereich angeordnet sein. Der stabförmige Sondenkörper steht dabei senkrecht zu einem zentralen Punkt des "Schalenbodens", wobei die Schale in Einführrichtung bzw. zu der Sonde hin geöffnet ist. Durch eine solche Ausbildung des Mikrophonhalters vereinfacht sich das Positionieren von einem oder mehreren Referenzmikrophonen.

Gemäß einer Ausführungsform ist das Referenzmikrophon zur Messung von Umgebungslärm an einer separaten Auswerteeinheit angeordnet. Die separate Auswerteeinheit kann Bauteile wie eine Anzeige aufweisen, für die in bzw. an der Sonde wenig Platz vorhanden wäre. Die Sonde kann mit der Auswerteeinheit z.B. drahtlos oder über eine Kabelverbindung kommunizieren. Das Referenzmikrophon zur Aufnahme des Umgebungslärms kann praktischerweise an dieser Auswerteeinheit angeordnet sein, so dass es beim Einführen der Sonde möglichst wenig stört und bei der Messung z.B. nicht durch einen Finger verdeckt wird. Zur Verwendung der Sonde werden folgende Schritte durchgeführt:
- teilweises Einführen einer Sonde in den Gehörgang eines Probanden,
- aktives Schalldämpfen von Umgebungslärm mittels der Sonde in einem Raumbereich außerhalb des Gehörgangs,
- Anregung otoakustischer Emission innerhalb des Gehörgangs und
- Messung otoakustischer Emission innerhalb des Gehörgangs.
Es wird eine Sonde verwendet, die es ermöglicht, sowohl OAE anzuregen und zu messen als auch gleichzeitig Umgebungslärm in einem Raumbereich am Gehörgangseingang durch aktive Schalldämpfung zu reduzieren. Das Einführen der Sonde ist kein invasiver Eingriff, sondern die Sonde wird lediglich in den Gehörgang eingeführt, ohne den Probanden (z.B. das Neugeborene bzw. den Patienten) zu verletzen. Dazu kann insbesondere eine der erfindungsgemäßen Sonden verwendet werden. Weiterhin kann bei diesem Vorgehen der Schritt durchgeführt werden, ein Fehlermikrophon zur aktiven Schalldämpfung oder einen Fehlermikrophoneingang, der über einen Sondenkanal an ein Fehlermikrophon angekoppelt ist, in einem Raumbereich am Gehörgangseingang anzuordnen. Durch die Anordnung des Fehlermikrophons oder des Fehlermikrophoneingangs an diesem Ort wird der Raumbereich der besten Auslöschung durch die aktive Schalldämpfung bestimmt. Bevorzugt wird der Umgebungslärm am Gehörgangseingang ausgelöscht.
In einem Ausführungsbeispiel des Verfahrens werden OAE mit Frequenzen kleiner als 1kHz gemessen.

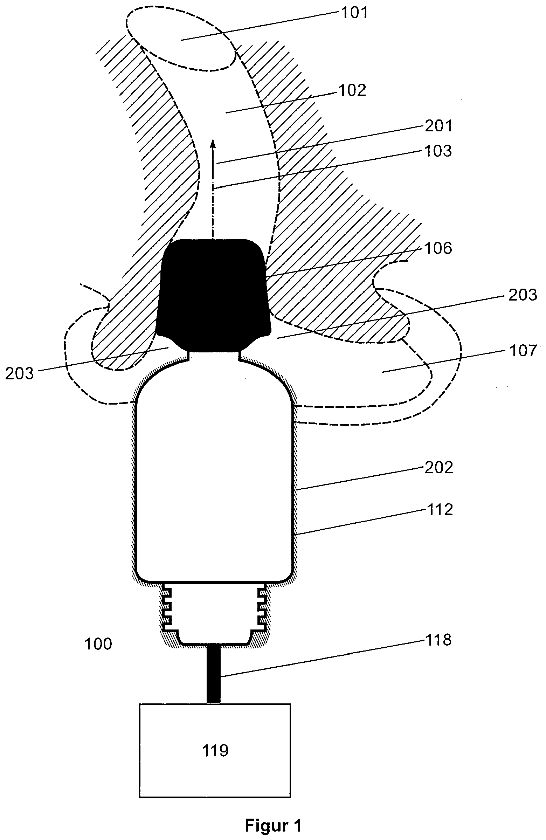

Ein Aspekt der Erfindung betrifft das Verwenden einer erfindungsgemäßen Sonde zur Messung von OAE.
Die Erfindung wird nachfolgend anhand von Figuren näher erläutert. Dabei sind einzelne Aspekte der Erfindung beispielhaft in den Figuren 1, 2 und 3 gezeigt und können mit anderen Ausführungsformen kombiniert werden. Gleiche oder ähnliche Merkmale der unterschiedlichen Ausführungsbeispiele weisen gleiche Bezugszeichen auf. Es zeigen:
- Figur 1:: eine schematische Darstellung einer in einen Gehörgang eingeführten Sonde zur Messung von OAE;
- Figur 2:: in einer schematischen Darstellung einen Querschnitt durch eine Sonde zur Messung von OAE mit im Sondenkörper integrierten Lautsprechern zur aktiven Schalldämpfung und Fehlermikrophonen, die über Sondenkanäle den Schalldruck messen; und
- Figur 3:: in einer schematischen Darstellung einen Querschnitt durch eine Sonde zur Messung von OAE mit in einen Sondenkörper integrierten Lautsprechern zur aktiven Schalldämpfung und Fehlermikrophonen, die ohne Sondenkanäle den Schalldruck messen.

Figur 1 zeigt in schematischer Darstellung eine Sonde 100 mit einem Sondenkörper 112 zur Messung von OAE. Die Sonde 100 befindet sich in der in Figur 1 gezeigten Position in Messposition bzw. Einführposition, in der die Sonde 100 in ein Ohr eingeführt ist. Dazu weist die Sonde 100 einen Einführbereich 106 auf, mit dem die Sonde 100 in den Gehörgang 102 des Ohres eingeführt ist.

Der Einführbereich 106 der Sonde 100 kann als Ohrstöpsel ausgebildet sein. Die Größe des Ohrstöpsels, insbesondere seine Tiefe und sein Durchmesser, sind auf die Gehörgangsgröße bzw. den Gehörgangsdurchmesser angepasst, so dass der Ohrstöpsel 106 den Gehörgang 102 im Messzustand leicht aufweitet und der Ohrstöpsel 106 in Messposition der Sonde 100 an seinem Mantelbereich mit dem Gehörgang 102 in Berührkontakt ist.

Die Sonde 100 ist im Wesentlichen zylinderförmig oder stabförmig ausgebildet, wobei die Zylinderlänge in Richtung einer Mittelachse 103 ausgerichtet ist.

In Messposition ist die Mittelachse 103 der Sonde 100 so ausgerichtet, dass die Sonde 100 aus dem Gehörgang 102 des Ohrs herauszeigt. Die Mittelachse 103 der Sonde 100 fällt mit einer Einführrichtung 201 der Sonde 100 zusammen. Mittelachse 103 und Einführrichtung 201 sind parallel zueinander.

Die Sonde 100 weist neben dem Einführbereich 106 einen externen Sondenbereich 202 auf, der in Messposition der Sonde 100 außerhalb des Ohres und des Gehörgangs 102 angeordnet ist.

Figur 1 zeigt weiterhin ein Trommelfell 101, zu dem der Gehörgang 102 führt, und eine Ohrmuschel 107 eines Probanden. Ein Gehörgangseingang 203 bezeichnet einen Bereich an der Ohrmuschel 107, der benachbart zum Gehörgang 102 angeordnet ist.

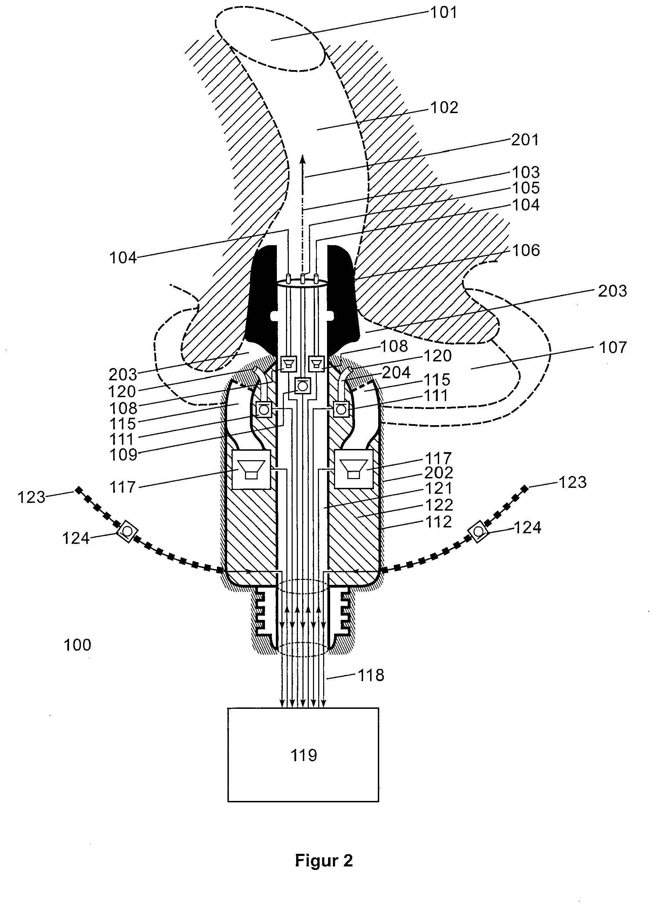

Figur 2 zeigt in schematischer Darstellung einen Querschnitt durch die Sonde 100 aus Figur 1. Insbesondere zeigt Figur 2 den inneren Aufbau der Sonde 100 mit einem Sondenkörper 112 zur Messung von OAE. Eine erste Gehäusekammer 121 der Sonde 100 verläuft durch den Sondenkörper 112 entlang der Mittelachse 103. Die erste Gehäusekammer 121 beinhaltet die Bestandteile der Sonde, die zur Anregung und Messung der OAE ausgebildet sind. Die Sonde weist weiterhin eine zweite Gehäusekammer 122 auf, die im externen Sondenbereich 202 die erste Gehäusekammer 121 umgibt. Die zweite Gehäusekammer 122 weist die Dämpfungsmittel der Sonde 100 auf, die für die aktive Schalldämpfung erforderlich und insbesondere zur Erzeugung eines Gegenschalls ausgebildet sind.

Die erste Gehäusekammer 121 weist ein Anregungsmittel 108 auf, das als Lautsprecher ausgebildet sein kann und an einen Sondenkanal gekoppelt sein kann. Ein Sondenausgang des Anregungsmittels 108 bzw. des angekoppelten Sondenkanals ist in Figur 2 als Anregungsmittelausgang 104 gezeigt. Der Anregungsmittelausgang 104 ist im Inneren des Gehörgangs 102 angeordnet, so dass das Anregungsmittel 108 zur Anregung von OAE verwendet werden kann.

Die erste Gehäusekammer 121 weist weiterhin ein Messmittel 109 zur Messung der OAE auf, das als Mikrophon bzw. Sondenmikrophon ausgebildet sein kann. Das Messmittel 109 kann an einen Sondenkanal mit einem Messmitteleingang 105 gekoppelt sein. Wie der Anregungsmittelausgang 104 ist der Messmitteleingang 105 des Messmittels 109 in Messposition der Sonde 100 im Inneren des Gehörgangs 102 angeordnet. Ein Mikrophon des Messmittels 109 kann dabei weiter im Inneren der Gehäusekammer 121 angeordnet sein. Die im Gehörgang 102 nach Anregung durch das Anregungsmittel 108 vorhandenen OAE treffen auf den Messmitteleingang 105 und werden über einen Sondenkanal zum Messmittel 109 geführt.

Zur Messung von OAE sendet das Anregungsmittel 108 z.B. Klicks für eine transitorisch evozierte otoakustische Emission oder zwei Sinustöne mit aufeinander abgestimmten Frequenzen zur Anregung einer distorsivproduzierten otoakustischen Emission aus. Im Innenohr erfolgt eine Reaktion auf diese Stimulation und das Innenohr sendet eine otoakustische Emission aus. Die otoakustische Emission wird vom Messmittel 109 gemessen.

Der Lautsprecher des Anregungsmittels 108 kann dabei weiter im Inneren der Gehäusekammer 121 angeordnet sein, der Schall des Lautsprechers wird jedoch so geführt, dass er im Inneren des Gehörgangs 102 aus der Sonde austritt.

Die Sonde 100 weist weiterhin eine Zuleitung 118 auf, die mit einer Auswerteeinheit 119 verbunden sein kann. Die Auswerteeinheit 119 kann sowohl zur Berechnung des Anregungssignals, zur Messung und Auswertung der OAE als auch zur Berechnung des Gegenschalls dienen. Die Auswerteeinheit 119 kann einen Mikroprozessor, insbesondere einen Computer mit entsprechender Software umfassen.

Die zweite Gehäusekammer 122 ist im Wesentlichen zylinderförmig aufgebaut und wird von der ersten Gehäusekammer 121 entlang der Zylinderachse, die mit der Mittelachse 103 zusammenfällt, durchdrungen.

Die Sonde 100 weist dabei zumindest jeweils ein Fehlermikrophon 111 und einen Lautsprecher 117 als Dämpfungsmittel auf und kann zusätzlich zumindest ein Referenzmikrophon 124 aufweisen.

An der zweiten Gehäusekammer 122 kann ein Mikrophonhalter 123 angeordnet sein, wie in Figur 2 gezeigt. Alternativ kann der Mikrophonhalter 123 auch an einem anderen Teil des Sondenkörpers 112 angeordnet sein. Der Mikrophonhalter 123 dient zum Positionieren eines oder mehrerer Referenzmikrophone 124. Der Mikrophonhalter 123 kann dazu Vorrichtungen aufweisen, in die das oder die Referenzmikrophone 124 eingeführt und befestigt werden können und aus einem flexiblen Material ausgebildet sein.

Die zweite Gehäusekammer 122 kann die Dämpfungsmittel für die aktive Schalldämpfung aufweisen. Das Dämpfungsmittel enthält den Lautsprecher 117, der als Miniaturlautsprecher mit Abmessungen unter 10mm ausgebildet sein kann. An dem Schallausgang der Lautsprecher 117 sind Schallführungen 115 angeordnet, die den von den Lautsprechern 117 emittierten Schall in Richtung zum Gehörgangseingang 203 weiterleiten. Die Ausgangsseite der Schallführung 115 ist dem Gehörgangseingang 203 zugewandt.

Das Fehlermikrophon 111 ist in der zweiten Gehäusekammer 122 angeordnet. In dem in Figur 2 gezeigten Ausführungsbeispiel ist das Fehlermikrophon 111 über einen Fehlermikrophonkanal 204 mit einem Fehlermikrophoneingang 120 verbunden. Der Fehlermikrophoneingang 120 ist in Messposition der Sonde 100 im Bereich des Gehörgangseingangs 203 eines Probanden angeordnet.

Das Fehlermikrophon 111 für die aktive Schalldämpfung kann als Mikrophon bzw. Sondenmikrophon mit Fehlermikrophonkanal 204 ausgebildet sein (vgl. Fig. 2).

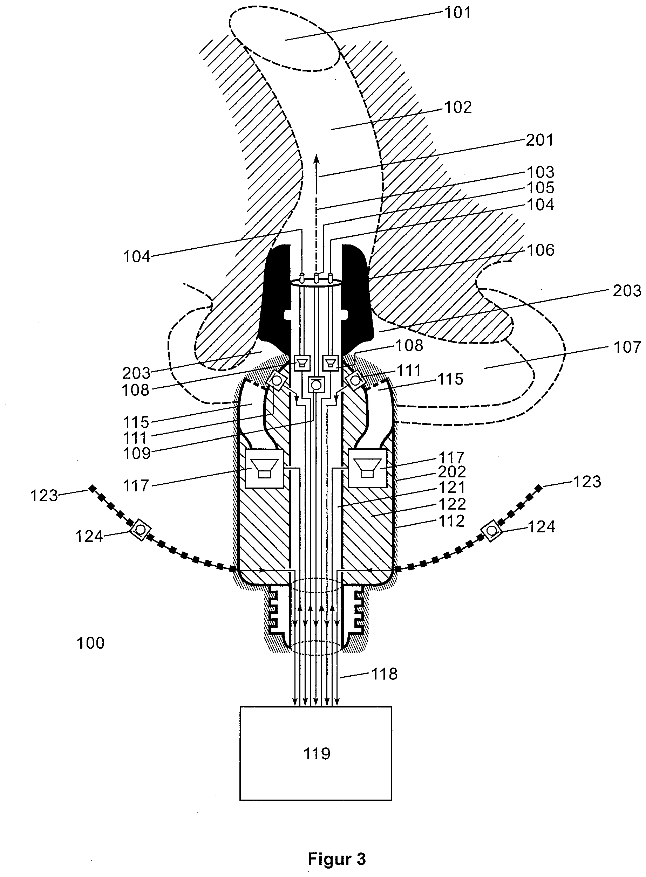

Bei dem in Figur 3 gezeigten Ausführungsbeispiel ist an dem Fehlermikrophon 111 kein Sondenkanal angekoppelt, das Fehlermikrophon 111 ist kanalfrei ausgebildet. Das bedeutet, dass das Fehlermikrophon 111 so in der zweiten Gehäusekammer 122 angeordnet ist, dass im Messzustand der Sonde 100 ein Detektionseingang des Fehlermikrophons 111 dem Gehörgangseingang 203 zugewandt ist und/oder in dem Gehörgangseingang 203 angeordnet ist.

Der Gehörgangseingang 203 ist dabei an einer Ohrmuschel 107 vor dem Gehörgang 102 des Probanden angeordnet.

Mit Hilfe des von dem oder den Referenzmikrophonen 124 und Fehlermikrophonen 111 (Figur 2 und Figur 3) erfassten Schalldrücken berechnet ein in der Auswerteeinheit 119 implementierter Algorithmus den Gegenschall, der über den bzw. die Lautsprecher 117 abgestrahlt wird und sich im Bereich des oder der Fehlermikrophone 111 bzw. der Fehlermikrophoneingänge 120 der Fehlermikrophone 111 mit Umgebungslärm destruktiv überlagert. Im Bereich des Fehlermikrophons 111 (Figur 3) bzw. im Bereich der Fehlermikrophoneingänge 120 des Fehlermikrophons 111 (Figur 2) entsteht dadurch eine schallgedämpfte Zone im äußeren Sondenbereich bzw. im Bereich am Gehörgangseingang 203.

Ein Konflikt zwischen der aktiven Schalldämpfung und der Erfassung der OAE wird durch die räumliche Trennung der Lautsprecher 117, der Fehlermikrophone 111 und ggf. der Referenzmikrophone 124 zur aktiven Schalldämpfung einerseits und der Anregungsmittel 108 und Messmittel 109 der OAE andererseits vermieden. Die aktive Schalldämpfung greift nicht in das Schallfeld im Gehörgang 102 ein, sondern dämpft den Umgebungslärm im äußeren Sondenbereich und damit am Gehörgangseingang 203, der durch den Ohrstöpsel 106 verschlossen ist.

Die OAE treten nur im Gehörgang 102 auf und werden daher nicht in die Berechnung des Gegenschalls einbezogen. Da die Interaktion zwischen Umgebungslärm und Gegenschall außerhalb des Gehörgangs 102 erfolgt, ist eine direkte Wirkung des Gegenschalls auf die OAE beinahe ausgeschlossen. Restschall, der trotzdem in den Gehörgang 102 eindringt, ist prinzipiell nicht anders zu betrachten als der Umgebungslärm, der bei einer Messung der OAE nach dem Stand der Technik in den Gehörgang 102 eindringt. Der wesentliche Unterschied besteht jedoch darin, dass die Intensität des Restschalls nach aktiver Schalldämpfung wesentlich gemindert ist, was die Auswertung der OAE wesentlich vereinfacht und eine Messung im Frequenzbereich unterhalb von 1 kHz ermöglicht.

Da zu erwarten ist, dass Umgebungslärm hauptsächlich durch den Ohrstöpsel eingekoppelt wird, bietet es sich an, das oder die Fehlermikrophone 111 (vgl. Figur 3) bzw. die Fehlermikrophoneingänge 120 der Fehlermikrophone 111 (vgl. Figur 2) in diesem Bereich zu positionieren. Durch die Schallführung 115 erfährt der von dem Lautsprecher 117 ausgesandte Gegenschall die gleiche Ausbreitungsrichtung wie der zum Gehörgangseingang 203 propagierende Umgebungslärm. Dazu ist die Positionierung der Schallführung 115 und des Fehlermikrophons 111 bzw. des Fehlermikrophoneingangs 120 des Fehlermikrophons 111 sowie ggf. die Positionierung des Referenzmikrophons 124 aufeinander abgestimmt. Diese Anordnung bewirkt, dass nach der Überlagerung des Umgebungslärms mit dem Gegenschall im Bereich des Fehlermikrophons 111 bzw. des Fehlermikrophoneingangs 120 eine deutlich gedämpfte Schallwelle in Richtung der Ohrmuschel 107 bzw. des Gehörgangseingangs 203 propagiert, die durch passive Dämpfungseigenschaften des Ohrstöpsels weiter gedämpft werden kann. Dazu ist der Einführbereich 106 aus einem Material mit guten passiven Dämpfungseigenschaften ausgebildet.

### Bezugszeichenliste

- 100: Sonde
- 101: Trommelfell
- 102: Gehörgang
- 103: Mittelachse
- 104: Anregungsmittelausgang
- 105: Messmitteleingang
- 106: Einführbereich
- 107: Ohrmuschel
- 108: Anregungsmittel
- 109: Messmittel

- 111: Fehlermikrophon
- 112: Sondenkörper

- 115: Schallführung

- 117: Lautsprecher
- 118: Zuleitung
- 119: Auswerteeinheit
- 120: Fehlermikrophoneingang
- 121: erste Gehäusekammer
- 122: zweite Gehäusekammer
- 123: Mikrophonhalter
- 124: Referenzmikrophon
- 201: Einführrichtung
- 202: externer Sondenbereich
- 203: Gehörgangseingang
- 204: Fehlermikrophonkanal

## Patentansprüche

1. Sonde zur Messung von otoakustischer Emission mit zumindest einem Referenzmikrophon (124) zur Messung von Umgebungslärm und mit einem Sondenkörper (112), wobei der Sondenkörper (112) teilweise entlang einer Einführrichtung (201) in einen Gehörgang (102) eines Probanden einführbar ausgebildet ist und der Sondenkörper (112) aufweist:
- einen Einführbereich (106), der zum Einführen in den Gehörgang ausgebildet ist, und einen externen Sondenbereich (202) aufweist, der dazu ausgebildet ist, beim Messen otoakustischer Emission außerhalb des Gehörgangs (102) angeordnet zu sein,
- ein Anregungsmittel (108) zur Anregung otoakustischer Emission und ein Messmittel (109) zur Messung der otoakustischen Emission, wobei das Anregungsmittel (108) und das Messmittel (109) im oder am Einführbereich (106) angeordnet sind und
- ein Dämpfungsmittel (117) zur aktiven Schalldämpfung, wobei das Dämpfungsmittel (117) im oder am externen Sondenbereich (202) ausgebildet ist,
wobei an der Sonde (100)
- als Dämpfungsmittel zumindest ein Lautsprecher (117) zum Aussenden von Gegenschall und
- zumindest ein Fehlermikrophon (111) zur Messung eines resultierenden Schalldrucks, der sich aus der Überlagerung des Umgebungslärms und des Gegenschalls ergibt,
so angeordnet sind, dass zum Fehlermikrophon (111) hin propagierender Schall aktiv gedämpft wird, wobei der Lautsprecher (117) und das Referenzmikrophon (124) in oder an einem externen Sondenbereich (202) angeordnet sind, **gekennzeichnet dadurch, dass** das Fehlermikrophon (111) zur aktiven Schalldämpfung an der Sonde (100) so angeordnet ist, dass das Fehlermikrophon (111) und ein Detektionseingang des Fehlermikrophons (111) oder ein Fehlermikrophoneingang (120) eines Fehlermikrophonkanals (204), der an das Fehlermikrophon (111) gekoppelt ist, im Einführzustand der Sonde (100) in einem Raumbereich vor dem Gehörgangseingang (203) angeordnet ist.

2. Sonde nach Anspruch 1, wobei das Dämpfungsmittel (117) in einer separaten Gehäusekammer (122) des Sondenkörpers (112) angeordnet ist.

3. Sonde nach einem der vorangegangenen Ansprüche, wobei das Fehlermikrophon (111) in oder an dem externen Sondenbereich (202) angeordnet ist.

4. Sonde nach einem der Ansprüche 1 oder 2, wobei das Fehlermikrophon (111) zur aktiven Schalldämpfung benachbart zum Messmittel (109) im Inneren der Sonde (100) angeordnet ist.

5. Sonde nach einem der vorangegangenen Ansprüche, wobei das Anregungsmittel (108) und das Messmittel (109) dazu ausgebildet sind, otoakustische Emission mit Frequenzen kleiner als 1 kHz zu messen.

6. Sonde nach einem der vorangegangenen Ansprüche, wobei am Sondenkörper (112) eine Schallführung (115) so angeordnet ist, dass vom Dämpfungsmittel (117) ausgesandter Gegenschall im Wesentlichen in Einführrichtung (201) geführt wird.

7. Sonde nach einem der vorangegangenen Ansprüche, wobei am Sondenkörper (112) ein Griffbereich ausgebildet ist, an dem die Sonde (100) beim Messen otoakustischer Emission festhaltbar ist.

8. Sonde nach einem der vorangegangenen Ansprüche, wobei ein Referenzmikrophon (124) zur Messung von Umgebungslärm an einem schalldurchlässigen Mikrophonhalter (123) angeordnet ist.

9. Sonde nach Anspruch 8, wobei der Mikrophonhalter (123) am Sondenkörper (112) angeordnet ist.

10. Sonde nach einem der Ansprüche 1 bis 8, wobei das Referenzmikrophon (124) zur Messung von Umgebungslärm an einer separaten Auswerteeinheit (119) angeordnet ist.

## Claims

1. A probe for measuring otoacoustic emission with at least one reference microphone (124) for measuring ambient noise and with a probe body (112), wherein the probe body (112) is formed to be partially insertable into an ear canal (102) of a test person along an insertion direction (201) and the probe body (112) comprises:
- an insertion region (106) adapted for insertion into the ear canal and having an external probe region (202) adapted to be located outside the ear canal (102) when measuring otoacoustic emission,
- an excitation means (108) for exciting otoacoustic emission and a measuring means (109) for measuring the otoacoustic emission, wherein the excitation means (108) and the measuring means (109) are arranged in or on the insertion region (106), and
- a attenuation means (117) for active sound attenuation, wherein the attenuation means (117) is formed in or on the external probe region (202), wherein on the probe (100)
- as an attenuation means, at least one loudspeaker (117) for emitting counter sound and
- at least one error microphone (111) for measuring a resulting sound pressure, which results from the superimposition of the ambient noise and the counter sound,
are arranged so that sound propagating to the error microphone (111) is attenuated actively, wherein the loudspeaker (117) and the reference microphone (124) are arranged in or on an external probe region (202), **characterized in that**
the error microphone (111) for active sound attenuation is disposed at the probe (100) so that the error microphone (111) and a detection input of the error microphone (111) or an error microphone input (120) of an error microphone channel (204) coupled to the error microphone (111) is arranged in a spatial region in front of the ear canal entrance (203) in the insertion state of the probe (100).

2. The probe according to claim 1, wherein the attenuation means (117) is arranged in a separate housing chamber (122) of the probe body (112).

3. The probe according to one of the preceding claims, wherein the error microphone (111) is arranged in or on the external probe region (202).

4. The probe according to one of claims 1 or 2, wherein the error microphone (111) for active sound attenuation is arranged adjacent to the measuring means (109) in the interior of the probe (100).

5. The probe according to one of the preceding claims, wherein the excitation means (108) and the measuring means (109) are adapted to measure otoacoustic emission with frequencies less than 1 kHz.

6. The probe according to one of the preceding claims, wherein on the probe body (112) a sound guide (115) is arranged so that counter sound emitted from the attenuation means (117) is guided substantially in the insertion direction (201).

7. The probe according to one of the preceding claims, wherein on the probe body (112) a grip region is formed, on which the probe (100) is fixable when measuring otoacoustic emission.

8. The probe according to one of the preceding claims, wherein a reference microphone (124) for measuring ambient noise is arranged on a sound-transmitting microphone holder (123).

9. The probe according to claim 8, wherein the microphone holder (123) is arranged on the probe body (112).

10. The probe according to one of claims 1 to 8, wherein the reference microphone (124) for measuring ambient noise is arranged on a separate evaluation unit (119).

## Revendications

1. Sonde pour la mesure d'une émission oto-acoustique avec au moins un microphone de référence (124) pour la mesure du bruit ambiant et avec un corps de sonde (112), sachant que le corps de sonde (112) est en partie réalisé de manière insérable le long d'une direction d'insertion (201) dans un canal auditif (102) d'un sujet et que le corps de sonde (112) présente :
- une zone d'insertion (106), qui est réalisée pour l'insertion dans le canal auditif, et qui présente une zone extérieure de sonde (202), qui est réalisée pour être disposée à l'extérieur du canal auditif (102) lors d'une mesure d'une émission oto-acoustique,
- un moyen d'excitation (108) pour exciter une émission oto-acoustique, et un moyen de mesure (109) pour la mesure de l'émission oto-acoustique, sachant que le moyen d'excitation (108) et le moyen de mesure (109) sont disposés dans ou auprès de la zone d'insertion (106), et
- un moyen d'atténuation (117) pour une atténuation acoustique active, sachant que le moyen d'atténuation (117) est réalisé dans ou auprès de la zone extérieure de sonde (202),
sachant qu'auprès de la sonde (100) sont disposés :
- comme moyen d'atténuation au moins un haut-parleur (117) pour l'émission de contre-sons ou encore d'un antibruit et
- au moins un microphone d'erreur (111) pour la mesure d'une pression acoustique résultante, qui est obtenu par la superposition du bruit ambiant et de l'antibruit,
de manière que des sons qui se propagent en direction du microphone d'erreur (111) sont atténués de manière active, sachant que le haut-parleur (117) et le microphone de référence (124) sont agencés dans ou auprès d'une zone extérieure de sonde (202), **caractérisée en ce que**
le microphone d'erreur (111) pour une atténuation acoustique active est agencé auprès de la sonde (100) de manière que le microphone d'erreur (111) et une entrée de détection du microphone d'erreur (111) ou une entrée de microphone d'erreur (120) d'un canal de microphone d'erreur (204), qui est couplée au microphone d'erreur (111), sont disposés dans une zone spatiale devant l'entrée du canal auditif (203) dans l'état d'insertion de la sonde (100).

2. Sonde d'après la revendication 1, sachant que le moyen d'atténuation (117) est agencé dans une chambre de boîtier (122) du corps de sonde (112) distincte.

3. Sonde d'après une des revendications précédentes, sachant que le microphone d'erreur (111) est agencé dans ou auprès de la zone extérieure de sonde (202).

4. Sonde d'après une des revendications 1 ou 2, sachant que le microphone d'erreur (111) pour une atténuation acoustique active est agencé à proximité immédiate des moyens de mesure (109) à l'intérieur de la sonde (100).

5. Sonde d'après une des revendications précédentes, sachant que le moyen d'excitation (108) et le moyen de mesure (109) sont réalisés pour mesurer des émissions oto-acoustiques avec des fréquences inférieures à 1 kHz.

6. Sonde d'après une des revendications précédentes, sachant qu'auprès du corps de sonde (112) est agencé un guidage acoustique (115) de manière qu'un contre-son émis par le moyen d'atténuation (117) est guidé essentiellement dans la direction d'insertion (201).

7. Sonde d'après une des revendications précédentes, sachant qu'auprès du corps de sonde (112) est réalisée une zone de préhension, par laquelle la sonde (100) peut être maintenue pendant la mesure d'une émission oto-acoustique.

8. Sonde d'après une des revendications précédentes, sachant qu'un microphone de référence (124) pour la mesure du bruit ambiant est disposé auprès d'un porte-microphone (123) acoustiquement transparent.

9. Sonde d'après la revendication 8, sachant que le porte-microphone (123) est disposé auprès du corps de sonde (112).

10. Sonde d'après une des revendications de 1 à 8, sachant que le microphone de référence (124) pour la mesure du bruit ambiant est disposé auprès d'une unité d'évaluation (119) distincte.
